Europäisches Patentamt

European Patent Office

Office européen des brevets

(19)

(11) Veröffentlichungsnummer: **0 204 203 B1**

# EUROPÄISCHE PATENTSCHRIFT

(45) Veröffentlichungstag der Patentschrift: **05.08.92**

(21) Anmeldenummer: **86106778.3**

(22) Anmeldetag: **17.05.86**

(51) Int. Cl.5: **A61F 6/14**, B21C 37/04, B23K 20/14, B32B 15/01, C23C 26/00

(54) **Verfahren zur Herstellung eines Drahtes aus zwei metallischen Werkstoffen.**

(30) Priorität: **03.06.85 DD 276883**

(43) Veröffentlichungstag der Anmeldung:
**10.12.86 Patentblatt 86/50**

(45) Bekanntmachung des Hinweises auf die Patenterteilung:
**05.08.92 Patentblatt 92/32**

(84) Benannte Vertragsstaaten:
**AT BE CH DE FR LI SE**

(56) Entgegenhaltungen:
DE-A- 2 153 318
US-A- 3 158 732

Dubbel, Taschenbuch f. den Maschinenbau, 14. Auflage, S.285

(73) Patentinhaber: **Medical-Produkte Lichtenberg GmbH**

**O-9208 Lichtenberg(DE)**

(72) Erfinder: **Gropp, Friedrich, Dipl.-Ing.**
**Muldaer Strasse 30**
**O-9208 Lichtenberg(DE)**
Erfinder: **Drechsler, Christoph**
**Dorfstrasse 106**
**O-9201 Burkersdorf(DE)**
Erfinder: **Müller, Rudolf**
**Strasse am Park 30**
**O-7062 Leipzig(DE)**
Erfinder: **Griesbach, Jürgen**
**Claussallee 43**
**O-9200 Freiberg(DE)**
Erfinder: **Sausmikat, Herbert**
**Dr.-Richard-Beck-Strasse 21**
**O-9200 Freiberg(DE)**

(74) Vertreter: **Zipse + Habersack**
**Kemnatenstrasse 49**
**W-8000 München 19(DE)**

# Beschreibung

Die Erfindung betrifft ein Verfahren zur Herstellung eines Kupferdrahtes mit einem Kerndraht aus Silber für die Verwendung in Intrauterinpessaren.

Es ist allgemein bekannt, daß beispielsweise die äußere Fläche einer in der Gebärmutter befindlichen Kupferspirale eine spermaabtötende Wirkung entwickelt, die bis zur Auflösung der gesamten Oberflächenschicht anhält. Zu dieser Wirkungsweise wird in der US-PS 3 563 235 erklärt, daß vom Kupfer eine physiologisch wirksame Ionenstrahlung ausgeht. Eine Gleichmäßigkeit dieser Strahlung über den Querschnitt des Uteruscavums wird nach der US-PS 3 200 815 am besten mit einer Spirale erreicht. Das Problem bei diesen intrauterinen Verhütungsmitteln lag jedoch im Zerbrechen der kupferabgebenden Spirale, die daraufhin mit einem Kerndraht aus einem anderen metallischen Werkstoff versehen wurde (DE-OS 22 07 939). Ihre Verfestigung wird durch Druck und höhere Temperaturen erreicht und erfolgt nach bekannten Diffusionsverfahren.

Die Vorgänge bei der Diffusion können natürlich zwischen Stoffen, die an den Berührungsflächen miteinander verbunden werden, wie z. B. im Diffusionssystem Kupfer und Zink, Kupfer und Silher oder Silber und Gold, ein unterschiedlich großes Diffusionsvermögen zeigen. Hierzu sei noch auf einen im Lehrbuch "Einführung in die Werkstoffkunde" VEB Verlag Technik Berlin, Ausgabe 1961 (Band 1, Allgemeine Metallkunde) veröffentlichten Abschnitt IX mit dem Titel "Die Diffusion in Metallen" verwiesen.

Die Erkenntnisse aus Diffusionsvorgängen zur Erlangung der benötigten Festigkeit zwischen zwei metallischen Werkstoffen können folglich auch dort genutzt werden, wo der Träger eines kontrazeptionsverhütenden Chemikals, z. B. Kupfer, auf ein anderes Metall, z. B. Silber, aufzubringen ist. Wenngleich verschiedene Arten von Vorrichtungen zur Kontrazeption als intrauterines Pessar vorgeschlagen worden sind, so z. B. die Anordnung der Kupferspirale auf dem flexiblen Kunststoffkörper oder die Verwendung von Metallringen kombinierter Metalle, die auf dem gemeinsamen Stiel des flexiblen Kunststoffkörpers aufgebracht sind, hat sich keiner als völlig zufriedenstellend erwiesen.

Wie einer Veröffentlichung in der US-Zeitschrift "Contraception", Los Altos 19 (1979) 1 entnommen werden kann, ist mit Hilfe einer derart geschaffenen Diffusionsschicht der Kupferüberzug der kontrazeptionsverhütenden Spirale auf einen korrosionsfesten Kerndraht aus Silber aufgefügt. Dieser hat eine Dicke, die 10 - 35 Prozent des Außendurchmessers des Kupferüberzuges beträgt, der seinerseits eine definierte Größe von 0,2 bis 0,4 mm aufweist. Mit dieser Größe des Außendurchmessers wird eine spermaabtötende Oberfläche der Kupferspirale geschaffen, wie diese auch schon durch die DE-OS 29 25 993 bekannt geworden ist.

Dieser zur Kupferspirale gewickelte Kupfer-Manteldraht mit Silberkern soll ein frühzeitiges Auflösen und Brechen desselben in der Gebärmutter durch die Diffusion der beiden Metalle vermeiden. Das Verfahren zur Herstellung einer dünnen Diffusionsschicht erfolgt in der Weise, daß aus reinem Kupfer ein Rohr von 46 mm/30 mm (Außendurchmesser/Innendurchmesser) gepreßt und dann auf eine Dicke von 20 mm/ 9 mm gezogen wird. Die Silberstange wird in einer Dicke von 12 mm gegossen und dann auf einen Durchmesser von 8,5 mm gezogen. Danach wird die Silberstange in die 9 mm weite Öffnung des Kupferrohres eingeführt und dann beide Werkstoffe gemeinsam auf einen Außendurchmesser von 18 mm gezogen. Im Anschluß erfolgt ein Glühen bei ca. 500 Grad Celsius, dessen Dauer ca. 20 Minuten beträgt. Bei dieser Temperatur findet erfahrungsgemäß noch keine Diffusion zwischen Kupfer und Silber statt.

In Fortsetzung dieses Verfahrens werden die Werkstoffe zunächst auf 12,8 mm Durchmesser gezogen und nochmals ca. 20 Minuten lang mit gleicher Temperatur geglüht, dann weiter auf 8 mm Durchmesser gezogen und danach ca. 20 Minuten lang bei 600 Grad Celsius diffusionsgeglüht. Anschließend wird der Draht durch zweimaliges Ziehen unter gleichzeitigem Weichmachen durch Widerstandsglühen auf das übliche Endmaß von 0,3 mm gezogen.

Das zur Anwendung kommende Widerstandsglühen ist zeitlich so begrenzt, daß es nicht zu besagtem Diffusionsvorgang kommt.

Eine Vorstellung zur beschriebenen mechanischen Technologie erfolgt beispielsweise im Fachbuch "Fließen und Kriechen der Metalle", Metall-Verlag GmbH, (1955) - WB. Mit diesem und anderen bekannten Verfahren können die beiden Metalle ein derart unterschiedliches Diffusionsvermögen erhalten, daß nicht selten durch zu rasches Abwandern der Atome des einen Metalls in das andere Metall die benötigte Festigkeit in der Berührungszone ausbleibt.

Die Erfindung stellt sich das Ziel, die aufgezeigten Nachteile zu vermeiden. Außerdem ist ein Gebrauchswertzuwachs zu erreichen, der insbesondere die klinische Wirksamkeit von Intrauterinpessaren verbessert.

Der Erfindung liegt die Aufgabe zugrunde, das Verfahren der eingangs beschriebenen Art so zu verändern, daß durch zusätzlich eingeführte Parameter eine bessere metallische Bindung bzw. größere Haftfestigkeit zwischen einem Kupferüberzug und einem Kerndraht aus Silber erreicht wird.

Diese Aufgabe wird erfindungsgemäß dadurch gelöst, daß zusätzlich und während mehrerer aufeinanderfolgender Ziehstufen die beiden den Draht bildenden Werkstoffe Kupfer und Silber unter Vakuum und bei einer Temperatur von 550 °C jeweils 35 Minuten lang zwischengeglüht werden und am Ende des gesamten Ziehprozesses der Draht einem Rekristallisationsglühen unter Vakuum bei 450 °C und einer Zeitdauer von 35 Minuten unterworfen wird.

Nach einem weiteren Vorschlag der Erfindung kann das Zwischenglühen jeweils mit Erreichen bestimmter Drahtdurchmesser erfolgen, die zweckmäßigerweise bei 25 mm, 11,5 mm, 4,5 mm und 0,8 mm festgelegt sind. Weiterhin wird vorgeschlagen, daß jeweils mit Erreichen der Drahtdurchmesser 4,5 mm und 0,8 mm und vor dem Zwischenglühen der Draht einer Dampfentfettung mit Trichloräthylen unterzogen wird. Das Trichloräthylen befindet sich dabei in einem Wärmebereich zwischen 87 und 95 °C.

Das erfindungsgemäße Verfahren bietet den Vorteil, daß außer einer besseren Haftfestigkeit zwischen Kupferüberzug und Kerndraht ein Diffusionsvermögen ohne Schwächung des Kerndrahtes erreicht wird. Dies schafft die Voraussetzung, daß der Kerndraht in fester Verbindung mit dem Kupferüberzug auch bei dessen Verwendung als kontrazeptionsverhütendes Chemikal bleibt. Das Verfahren zur Herstellung eines Drahtes aus zwei metallischen Werkstoffen, insbesondere eines Kupferdrahtes mit einem Kerndraht aus Silber, zeigt klar die Vorteile der erfindungsgemäßen Diffusion und auch die einfache Möglichkeit ihrer Realisierung.

Das Verfahren erfolgt in der Weise, daß der Kernwerkstoff aus Feinsilber Ag 99, 96 zunächst erschmolzen und als Bolzen unter Schutzgas gegossen, dann auf einen Durchmesser von 12,5 mm warm stanggepreßt und anschließend auf einen Stangendurchmesser von 11,8 mm kalt gezogen wird. Der aus reinem Kupfer SE-Cu 99, 95 bestehende Mantelwerkstoff wird zunächst als Kupferrohr vorgefertigt, indem ein stanggepreßter Bolzen von 50 mm Durchmesser nach dem Abdrehen seiner Oberfläche auf das Außenmaß von 45 mm mit einer durchgehenden Bohrung von 12,5 mm versehen wird.

Nach dem Einführen der Silberstange in das Kupferrohr werden die beiden Werkstoffe auf ein gemeinsames Außenprofil von 8 mm kaltgewalzt. Dazwischen und zwar jeweils mit Erreichen der Schlüsselweiten 25 mm und 11,5 mm, erfolgt ein Zwischenglühen unter Vakuum bei 550 °C und einer Glühzeit von 35 Minuten. Danach wird in fünf Zügen auf einen Durchmesser von 4,5 mm kaltgezogen und anschließend unter Vakuum bei gleicher Temperatur und Glühzeit zwischengeglüht.

Dieser Verfahrensstufe folgt eine weitere Verringerung des Drahtquerschnitts, indem in 15 Zügen und mit zweimaligem Zwischenglühen auf den Enddurchmesser von 0,35 mm kaltgezogen wird. Das Zwischenglühen wird wieder unter Vakuum bei gleicher Glühtemperatur von 550 °C und unter Zeitdauer von jeweils 35 Minuten durchgeführt, sobald der Draht einen Außendurchmesser von 2,0 mm und dann von 0,8 mm erreicht hat. Vor den Zwischenglühungen erfolgt eine Dampfentfettung in Trichloräthylen, dessen Temperatur 87 bis 95 °C beträgt.

Zum Abschluß wird unter Vakuum ein Rekristallisationsglühen bei einer Temperatur von 450 °C und in einer Glühzeit von 35 Minuten durchgeführt, welches den Herstellungsprozeß des Kupferdrahtes mit einem Kerndraht aus Silber beendet.

Die Diffusion erfolgt jeweils bei den einzelnen Zwischenglühungen, so daß am Enderzeugnis eine durch die Diffusionsvorgänge geschaffene Diffusionszone von ca. 4 $\mu$m vorhanden ist.

Mit dem erfindungsgemäßen Verfahren ist es möglich geworden, die bei Spiralen für Intrauterinpessare erforderliche Kupferdrahtqualität reproduzierbar zu gestalten.

Es sei zum Verfahren noch der Hinweis gegeben, daß vor dem Einführen der Silberstange in das Kupferrohr die beiden zu verbindenden Metallflächen äußerst sauber sein müssen, damit im Kontaktflächenbereich eine Diffusion der Elemente und Moleküle stattfinden kann.

**Patentansprüche**

1. Verfahren zur Herstellung eines Kupferdrahtes mit einem Kerndraht aus Silber, deren Berührungszone durch Diffusionsvorgänge verfestigt ist, für die Verwendung in Intrauterinpessaren, dadurch **gekennzeichnet**, daß zusätzlich und während mehrerer aufeinanderfolgender Ziehstufen die beiden Werkstoffe unter Vakuum und bei einer Temperatur von 550° C jeweils 35 Minuten lang zwischengeglüht werden und am Ende des gesamten Ziehprozessen der Draht einem Rekristallisationsglühen unter Vakuum bei 450 ° C und einer Zeitdauer von 35 Minuten unterworfen wird.

2. Verfahren nach Anspruch 1, dadurch **gekennzeichnet**, daß das Zwischenglühen jeweils mit Erreichen des Drahtdurchmessers 25 mm, 11,5 mm, 4,5 mm, 2,0 mm und 0,8 mm erfolgt.

3. Verfahren nach Anspruch 1 oder 2, dadurch **gekennzeichnet**, daß jeweils mit Erreichen der Drahtdurchmesser 2,0 mm und 0,8 mm und vor dem Zwischenglühen der Draht einer Dampfentfettung mit Trichloräthylen bei einer Temperatur von 87 bis 95 ° C unterzogen wird.

## Claims

1.  Method for producing a copper wire having a core wire made of silver, the respective contact region being strengthened by diffusion processes, for use in intra-uterine pessaries, **characterized** in that additionally to and during a plurality of successive drawing stages, both materials are respectively intermediately annealed for 35 minutes under vacuum pressure and at a temperature of 550 ° C, and that at the end of the entire drawing process, the wire is subjected to a recristallisation annealing for a time period of 35 minutes under vacuum pressure and at 450 ° C.

2.  Method of claim 1, **characterized** in that said intermediate annealing is conducted when the wire diameter reaches 25 mm, 11.5 mm, 4.5 mm, 2.0 mm and 0.8 mm, respectively.

3.  Method of claim 1 or 2, **characterized** in that, when the wire diameter reaches 2.0 mm and 0.8 mm, respectively, and prior to said intermediate annealing, the wire is subjected to steam degreasing by trichloroethylene at a temperature of from 87 to 95 ° C.

## Revendications

1.  Procédé de fabrication d'un fil de cuivre, avec un fil central en argent, dont la zone de contact est durcie par des procédés de diffusion, pour l'utilisation dans des pessaires intra-utérines, caractérisé en ce qu'en plus et pendant plusieurs étapes d'étirage successives, les deux matériaux sont soumis à un recuit intermédiaire, sous vide à une température de 550 ° C, chaque fois pendant 35 minutes, et, à la fin de l'ensemble du processus d'étirage, le fil est soumis à un recuit de recristallisation sous vide, à 450 ° C et pendant une durée de 35 minutes.

2.  Procédé selon la revendication 1, caractérisé en ce que le recuit intermédiaire s'effectue chaque fois que l'on atteint un diamètre de fil de 25mm, 11,5mm, 4,5mm, 2,0mm et 0,8mm.

3.  Procédé selon la revendication 1 ou 2, caractérisé en ce que chaque fois que l'on atteint un diamètre de fil de 2,0mm et 0,8mm, et avant le recuit intermédiaire, le fil est soumis à un dégraissage à la vapeur, à l'aide de trichloréthylène, à une température de 87 à 95 ° C.